# EUROPEAN PATENT APPLICATION

(11) **EP 4 194 044 A1**
(43) Date of publication of application: **14.06.2023**
(21) Application number: 22209367.6
(22) Date of filing: 24.11.2022
(51) Int. Cl.: A61M 29/00, A61F 2/04

(54) **IMPLANT AND HANDHELD MEDICAL DEVICE**

(30) Priority: 13.12.2021 US 202163288879 P
(71) Applicant: Olympus Winter & Ibe GmbH, 22045 Hamburg (DE)
(72) Inventor: Brockmann, Christian, 21279 Hollenstedt (DE); Jürgens, Thorsten, 20359 Hamburg (DE)
(74) Representative: Hoener, Matthias

(57) **Abstract**

The invention provides an implant and a hand-held medical device with which the implant can be removed particularly easily and without traumatising the patient. This is achieved in that a proximal end (14) of an implant (10) has a coupling piece (15, 17) with a ferromagnetic element (18), wherein the implant (10) can be pulled out of the urethra via this coupling piece (15, 27).

## Description

The invention relates to an implant according to the claim 1. Furthermore, the invention relates to a hand-held medical device according to claim 10.

Various methods and techniques are known for the treatment of *benign prostatic hyperplasia (BPH).* In a minimally invasive, particularly body-friendly treatment of BPH symptoms, a removable implant is temporarily placed in the urethra or in the prostatic part of the patient's urethra. Such an implant is a wire structure made of a shape memory alloy, such as nitinol. The wire structure is pushed into place by a catheter in a collapsed state, where it unfolds into its predetermined basic structure. This structure, which can be formed from three or four wires, is a basket structure. This basket structure expands the urethra in its unfolded basic structure. Due to the expansion of the wire structure against the tissue of the urethra, the stricture tissue of the urethra is denatured in the course of a few days. This denaturation of the tissue occurs due to the ischaemic pressure of the individual wires on the cells of the tissue, resulting in a reduced or complete lack of blood flow. As a result, the lack of blood flow leads to a lack of oxygen in the cells and finally to cell death. Within a few days, the tissue can be reduced to such an extent that the urine flow almost returns to normal. After completion of this treatment, the implant can be retrieved from the urethra by means of a catheter.

In the technique described above, the implant has a thread or suture at a proximal end. To retrieve the implant from the urethra, the catheter is passed over this thread and thus guided to the proximal end of the implant. By pulling on the thread, the implant is folded or pulled into the catheter. The wire structure folds up in such a way that it fits into the catheter. A disadvantage is that the thread remains in the urethra or protrudes from the urethra during the entire treatment phase. This is not only unpleasant, it can also lead to infections, due to which the treatment has to be discontinued.

On this basis, the invention is based on the problem of creating an implant as well as a hand-held medical device with which the implant can be removed particularly easily and without traumatising the patient.

A solution to this problem is described by the features of claim 1. Accordingly, it is provided that a proximal end of an implant comprises a coupling piece with a ferromagnetic element, the implant being extractable from the urethra via this coupling piece. This coupling piece is much shorter than the thread described above and can therefore be located entirely in the urethra. To retrieve the implant from the body, it is contacted inside the urethra by a hand-held medical device via the coupling piece and pulled out. During this contact or coupling between the coupling piece of the implant and the hand-held medical device, a magnetic force acts between the ferromagnetic element of the implant and the hand-held medical device. For this purpose, the hand-held medical device also has a corresponding ferromagnetic counter element or magnet at a distal end. Due to the ferromagnetic element of the implant, an attractive effect is formed between the coupling piece of the implant and the hand-held medical device. This makes it possible for the implant to be contacted or for the necessary coupling between the implant and the hand-held medical device to take place. The magnetic attraction serves, in particular, for a correct positioning of the coupling piece relative to the hand-held medical device. The ferromagnetic element within the coupling piece thus makes it possible to contact the implant and to retrieve it in a particularly simple and gentle way.

In particular, it is provided according to the invention that the ferromagnetic element is a permanent magnet. This permanent magnet causes the coupling piece of the implant to have an attractive effect on ferromagnetic elements in its vicinity. For example, a magnetic or magnetisable distal end of a handheld medical device is attracted to the permanent magnet. This permanent magnet may be in the form of a bar magnet and have a size of one or a few millimetres. The advantage of a permanent magnet is that the magnetic polarisation is fixed. The coupling piece of the implant can therefore be contacted with a corresponding ferromagnetic object so that an attractive effect is formed between the elements.

Furthermore, it can be provided according to the invention that the ferromagnetic element is the coupling piece itself. For this purpose, the coupling piece, which is connected to the implant, is designed as a ferromagnetic element or permanent magnet. Furthermore, it is conceivable that the element has a coating in order to be protected against external chemical influences. It is also conceivable that the ferromagnetic element is integrated into the coupling piece, in particular that it is enclosed by the coupling piece.

One embodiment could provide for a permanent magnet or ferromagnetic element to be overmoulded by a coupling piece. In this example, the coupling piece is made of a plastic which flows around the ferromagnetic element during manufacture and is formed in such a way that the magnetic field lines outside the coupling piece are still strong enough for the above-mentioned purposes.

Preferably, it is conceivable that the coupling piece has at least one projection, a slot, a notch, a bore, a pin or the like as a mechanical coupling means. In addition to the magnetic coupling, the implant may form a mechanical bond by positive locking with a corresponding counterpart of the hand-held medical device. For such a mechanical connection, the coupling piece may have at least one projection, a slot, a notch, a hole or a pin. It is conceivable, for example, that corresponding teeth or a slot or a projection or a hole or a pin engage mechanically with each other and are additionally reinforced or held in position by magnetic force. A particularly advantageous embodiment of the invention may provide that the implant is first positioned relative to the hand-held medical device by the magnetic effect, in order to then enter into the mechanical connection. The combined mechanical and magnetic force then allows the implant to be removed from the urethra in a gentle and simple manner.

Furthermore, it can be provided according to the invention that the coupling piece of the implant has a circular or circular section-like cross-section. This shape makes transport through the urethra particularly easy and gentle on the body. Any projections or edges could cause trauma to the patient and should therefore be avoided.

A particularly advantageous embodiment of the invention may provide that a sensor and/or a data memory are arranged in the coupling piece, in particular that a sensor and/or a data memory are integrated in the coupling piece. The implanted implant can be identified by means of this sensor and/or this data memory. For this purpose, corresponding identifiers or information are stored on the data memory, which can provide information about the implant and the patient. Information about the implant and the patient can be verified by contactless reading using a corresponding scanner or in contact with the handheld medical device. In addition, it is conceivable that the sensor, which may be a reed sensor or a metal sensor such as a Hall probe, can be used to detect whether the coupling of the coupling piece with a coupling counterpart has been established. Both the sensor and the data memory can be a passive component in order to keep the size as small as possible.

According to the invention, it is conceivable that the coupling piece is connected via a wire or a thread or directly to the wire structure of the implant. In particular, the length of the wire or thread should be kept as short as possible. While a direct connection of the coupling piece to the wire structure makes for a very compact implant, a wire or thread can be used to make the implant more flexible, which could ultimately be more comfortable for the patient. The length of the thread or even the wire between the coupling piece and the wire structure can be a few millimetres, in particular 1 mm to 5 mm or 1 mm to 10 mm, or 1 cm to 2 cm or even 1 cm to 3 cm. For recovering the implant, it may be advantageous if the coupling piece is connected to the wire structure via a thread or wire for coupling or positioning the coupling piece relative to a counterpart coupling piece. On the other hand, positioning the wire structure is easier if the coupling piece is directly connected to the wire structure. Then the wire structure can be moved directly into the correct position via the coupling piece.

A hand-held medical device for solving the above-mentioned problem has the features of claim 10. Accordingly, it is provided that a hand-held medical device for positioning and retrieving an implant in a urethra or from a urethra has a rod, in particular a flexible rod. This rod can be inserted into the urethra via a catheter. At a distal end, the rod has a coupling counterpart. The implant is designed according to at least one of claims 1 to 9. The essential feature of the handheld medical device according to the invention is that the coupling counterpart comprises a ferromagnetic counterpart for coupling with a coupling piece of the implant. This hand-held medical device allows the implant to be contacted and removed from the human body in a very reliable and gentle manner. Due to the ferromagnetic design of the counter element, a guided coupling or positioning of the coupling counter piece with the coupling piece takes place. Due to the magnetic interaction between the implant and the handpiece, the treating person only has to move the distal end of the rod close to the proximal end of the implant. The coupling counterpart and the coupling piece then fit into their coupling positions due to their attractive effect. Similarly, positioning the implant in the urethra is particularly advantageous. During insertion, the contact between the coupling piece and the counterpart or between the rod and the implant is strengthened by the magnetic interaction. Thus, there is not only a mechanical but also a magnetic connection between the implant and the rod, which enables a safe placement of the implant in the urethra. However, the magnetic field strength makes it possible to separate the two aforementioned pieces by applying a small amount of force or rotating the rod.

An advantageous further development of the handheld medical device may provide that the ferromagnetic counter element is a permanent magnet. This permanent magnet causes the coupling counterpart of the rod to have an attractive effect on the ferromagnetic element in its vicinity. For example, a magnetic or magnetisable distal end of the implant is attracted to the permanent magnet. This permanent magnet may be a bar magnet and have a size of one or a few millimetres. The advantage of a permanent magnet is that the magnetic polarisation is fixed. The coupling counterpart of the rod can therefore be contacted with a corresponding ferromagnetic object so that an attractive effect is formed between the elements.

Preferably, it can be further provided that the ferromagnetic counter element is the coupling counter piece. For this purpose, the coupling counterpart, which is connected to the rod, is designed as a ferromagnetic counterpart or permanent magnet. Furthermore, it is conceivable that the counter element has a coating in order to be protected against external chemical influences. Equally, however, it is also conceivable that the ferromagnetic counter element is integrated into the coupling counter piece, in particular is enclosed by the coupling counter piece. An embodiment example could provide that a permanent magnet or a ferromagnetic counter element is overmoulded by a coupling counter part. In this example, the coupling counterpart is made of a plastic which closes around the ferromagnetic counterpart element and is formed in such a way that the magnetic field lines outside the coupling counterpart are still strong enough for the above-mentioned purposes.

A particularly advantageous embodiment of the invention may provide that the coupling counterpart comprises at least one projection, slot, notch, hole, pin or the like as mechanical coupling means, said coupling means being complementary to the coupling means of the coupling piece. In addition to the magnetic coupling, the rod may form a mechanical bond by positive engagement with a corresponding counterpart of the implant. For such a mechanical connection, the coupling counterpart may comprise at least one projection, a slot, a notch, a bore and/or a pin. It is conceivable, for example, that corresponding teeth or a slot or a notch or a hole or a pin engage mechanically and are additionally reinforced or held in position by magnetic force.

A particularly advantageous embodiment of the invention may provide that the implant is first positioned relative to the handheld medical device by the magnetic effect in order to then enter into the mechanical connection. The combined mechanical and magnetic force then allows the implant to be retrieved from the urethra in a gentle and simple manner.

Furthermore, it can be provided according to the invention that the coupling piece of the implant and the coupling counterpart of the rod have a common circular or circle-like cross-section in the assembled state. This shape makes transport through the urethra particularly easy and gentle on the body. Any protrusions or edges could cause trauma to the patient and should therefore be avoided. This allows the coupling piece and the counterpart to be pulled into the catheter together. Inside the catheter, the coupling is thus secured against radial displacement. This allows pulling with higher force without unintentionally loosening the magnetic coupling.

A particularly advantageous embodiment of the invention may provide that a sensor and/or a data memory are arranged in the coupling counterpart, in particular that a sensor and/or a data memory are integrated in the coupling counterpart. The implanted implant can be identified by means of this sensor and/or this data memory. For this purpose, corresponding identifiers or information are stored on the data memory, which can provide information about the implant, the handheld device used and the patient. Information about the handheld device and the patient can be verified by contactless reading using a corresponding scanner or by contacting the handheld medical device. In addition, it is conceivable that the sensor, which may be a reed sensor or a metal sensor such as a Hall probe, can be used to detect whether the coupling of the coupling counterpart with the coupling piece has been established. Both the sensor and the data memory can be passive components in order to keep the size as small as possible.

A preferred embodiment of the present invention is explained in more detail below with reference to the drawing. In this show:

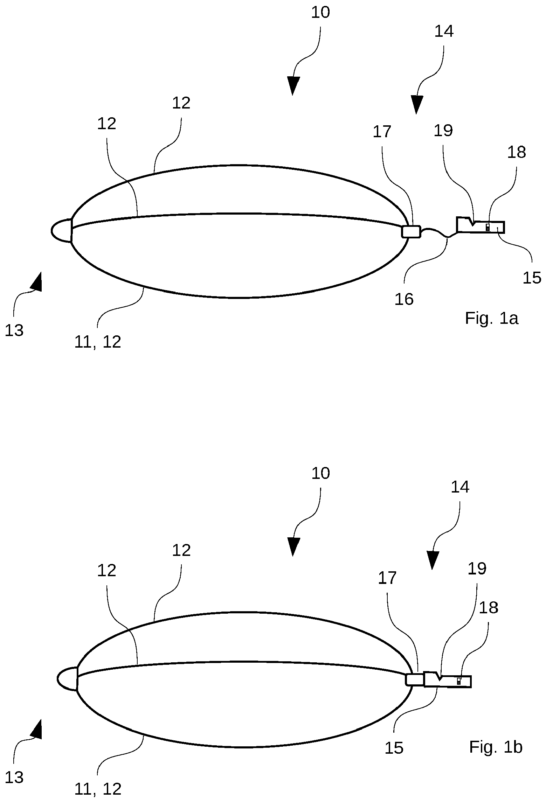

- Fig. 1a: a schematic representation of an implant,
- Fig. 1a: schematic representation of a further embodiment of an implant,
- Fig. 2: a schematic representation of a handheld medical device,
- Fig. 3a: schematic representation of an example of a coupling mechanism,

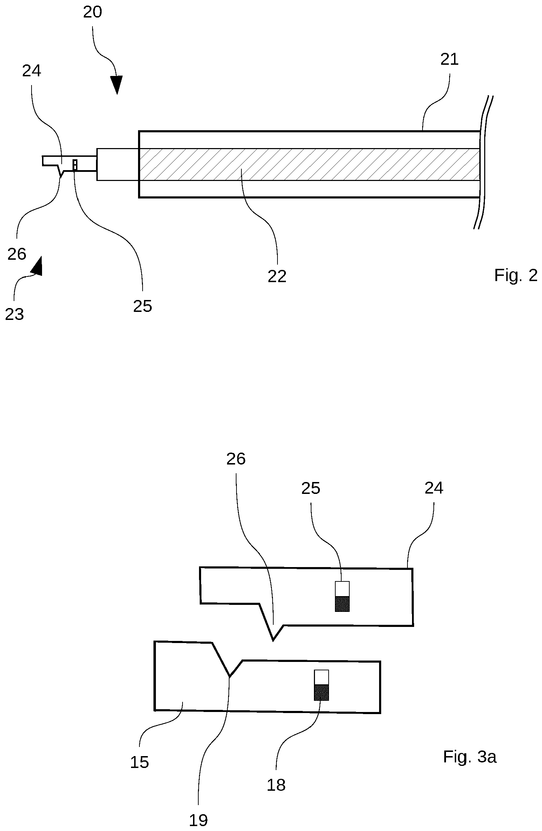

- Fig. 3a: further schematic representation of the coupling mechanism according to Fig. 3a,

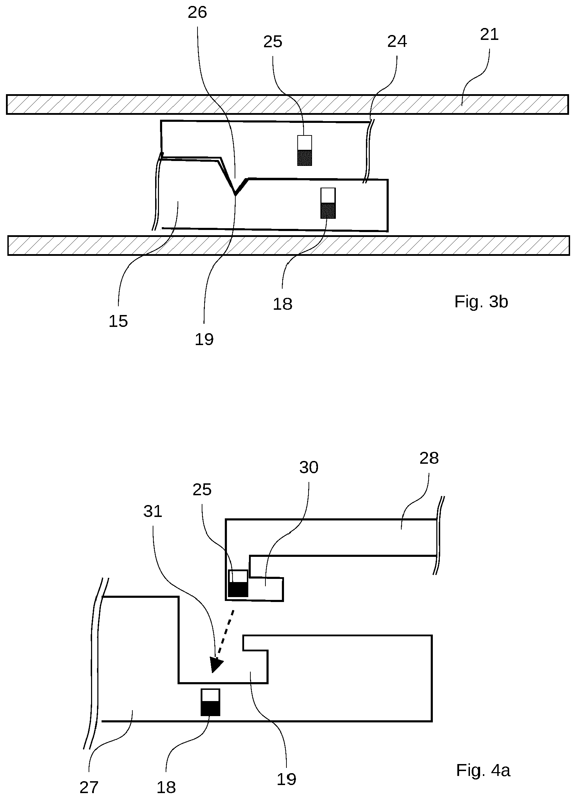

- Fig. 4a: a first schematic representation of a coupling process,

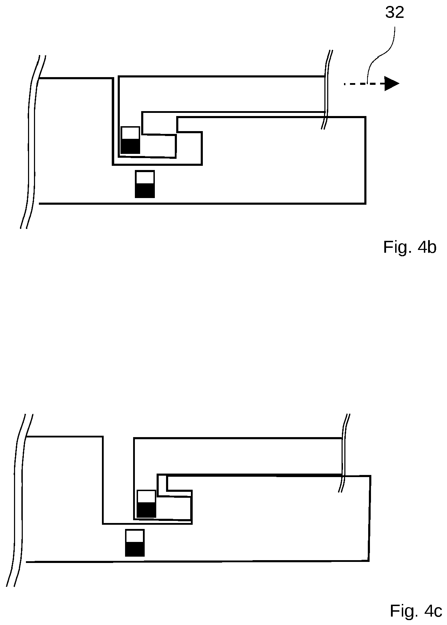

- Fig. 4b: a second schematic representation of the coupling process, and
- Fig. 4c: a third schematic representation of the coupling process.

The figures schematically illustrate possible embodiments of the invention. It should be expressly noted that the invention is not intended to be limited to these embodiments. Rather, it is intended that the invention can also be realised by other embodiments.

Fig. 1a shows a highly schematised representation of an implant 10. This implant 10 consists of a wire structure 11 consisting of three individual wires 12. These wires 12 are connected to each other at a distal end 13 and a proximal end 14 of the implant 10. The wires 12 are made of a shape memory material, such as nitinol.

For the treatment, the implant 10 is inserted into the patient's urethra in a folded state through a catheter not shown. At the correct position, the wires 12 unfold to form the basket structure shown in Fig. 1a. In addition to the structure shown here as an example, a variety of other shapes are conceivable. For a further description of the wire structure 11, reference is made to the prior art.

For placement and retrieval of the implant 10, it has a coupling piece 15 at the proximal end 14. This coupling piece 15 is connected to a proximal end piece 17 of the implant 10 via a thread 16. As an alternative to the thread 16, it is also conceivable to use a thin wire. The thread 16 is 1 mm to 10 mm or 5 mm long. In another embodiment, the coupling piece 15 is directly connected to the proximal end piece 17 (Fig. 1b).

The coupling piece 15 has a ferromagnetic element 18. This ferromagnetic element 18 may be a metal, in particular iron, nickel or cobalt or a magnetisable alloy, or a permanent magnet. A further embodiment of the invention may provide that the entire coupling piece 15 is formed as a ferromagnetic element 18 or as a permanent magnet. In the embodiment example shown in Fig. 1a, the ferromagnetic element 18 is formed as a bar magnet integrated in the coupling piece 15. This bar magnet is moulded into a plastic of the coupling piece 15, for example, during an injection moulding process. In this casting process, it is also conceivable that the thread 16 is also cast in. Alternatively, it is also conceivable that the thread 16 is subsequently connected to the coupling piece 15.

The embodiment of the coupling piece 15 shown in Fig. 1a and 1b also has a notch 19. This notch 19 serves as a mechanical coupling means. In addition to this notch 19 or as an alternative to this notch 19, the coupling piece 15 can also have a projection, a slot, a bore or a pin for mechanical coupling or contacting.

The implant 10 is coupled to a handheld medical device 20 for placement and/or retrieval. This hand-held medical device 20 is inserted into the urethra through a catheter 21, as shown schematically in Fig. 2. In this case, the hand-held medical device 20 essentially comprises a rod 22 which has a coupling counterpart 24 at a distal end 23. This coupling counterpart 24 is complementary to the coupling piece 15. Thus, the coupling counterpart 24 also has a ferromagnetic counter-element 25. This ferromagnetic counter-element 25 enters into a magnetic interaction with the ferromagnetic element 18 of the coupling piece 15. For this purpose, it can be designed as a magnetisable metal or as a permanent magnet. If the ferromagnetic element 18 and the ferromagnetic counter element 25 are both formed as permanent magnets, they are oriented in such a way that their polarisation is opposite. This allows an attractive magnetic interaction to form between the coupling piece 15 and the counterpart coupling piece 24.

Another embodiment may provide that the ferromagnetic counter element 25 is formed as an electromagnet which can be switched on and off via the rod 22. Furthermore, the polarisation of the electromagnet can be changed so that either an attractive or repulsive force can be exerted on the ferromagnetic element 18 or the coupling piece 15.

The coupling piece 24 also has a tooth-like projection 26. This tooth-like projection 26 is complementary to the notch 19 of the coupling piece 15. This projection 26 also represents only one of many possible embodiment examples. It is also conceivable here that, as an alternative to the projection 26, the coupling counterpart 24 has a slot, a bore, a notch, a pin or the like.

In Fig. 3a, for better illustration, the coupling piece 15 and the coupling counter piece 24 are shown side by side without the implant 10 or without the rod 22. In this position, the ferromagnetic element 18 and the ferromagnetic counter element 25, both stylised as permanent magnets, act attractively on each other. This attractive force helps the user of the coupling counterpart 24 to contact the coupling piece 15. As soon as these two pieces 15, 24 make contact with each other, a latching of the projection 26 with the notch 19 occurs. The permanent magnets hold this contact. In this coupling state, which is shown in a highly schematised form in Fig. 3b, the implant 10 can be placed in the urethra in a very simple and precise manner as well as recovered from the urethra through the catheter. The coupling piece 15 and the counterpart coupling piece 24 form a circular cross-section in the coupled state. This allows both to be pulled together into the catheter 21. Inside the catheter 21, the coupling is thus secured against radial displacement. This makes it possible to pull with greater force without unintentionally loosening the magnetic coupling.

Due to the low magnetic interaction between the two permanent magnets, the hand-held medical device 20 can be removed from the coupling piece 15 by applying a small amount of force or by twisting the rod 22 slightly after the implant 10 has been placed. To retrieve the implant 10, it is pulled out through the catheter by means of the hand-held medical device 20. In the process, the wire structure 11 folds up so that it fits into the catheter 21.

In order to make this procedure particularly gentle on the body, it is also provided that a common cross-section of the coupling piece 15 and the coupling counter-piece 24 is circular or circle-shaped. This allows adaptation to the anatomical conditions and placement or retrieval can be carried out without traumatising the urethra.

Figs. 4a to 4c show a further embodiment example for a coupling piece 27 and a coupling counter piece 28. For the sake of clarity, the implant 10 and the rod 22 are not shown here either. Exactly as previously described, both the coupling piece 27 and the coupling counter-piece 28 have a ferromagnetic element 18 and a ferromagnetic counter-element 25, both being designed as permanent magnets with an opposite polarisation. While the coupling piece 27 has an engagement 29, the coupling counterpart 28 has a corresponding hook structure 30, which corresponds in a complementary manner with the shape of the engagement 29.

As shown schematically in Fig. 4a, the coupling counterpart 28 is attracted to the coupling piece 27 in the direction of arrow 31 due to the magnetic interaction. As soon as the hook structure 30 has been brought close to or into engagement 29, the coupling piece 27 or the rod 22 is pulled in the proximal direction in the direction of the arrow 32 (Fig. 4b). This results in a mechanical as well as magnetic connection between the coupling piece 27 and the coupling counter piece 28 (Fig. 4c). In this constellation, the implant can be placed in the urethra and retrieved from the urethra in a very simple and gentle manner. To uncouple the coupling counterpart 28 from the coupling piece 27, the steps described above must be carried out in the opposite order.

### List of Reference Numerals:

- 10: Implant
- 11: Wire structure
- 12: Wire
- 13: Distal end
- 14: Proximal end
- 15: Coupling piece
- 16: Thread
- 17: End piece
- 18: Element
- 19: Notch
- 20: Hand-held medical device
- 21: Catheter
- 22: Rod
- 23: distal end
- 24: Coupling counterpart
- 25: Counter element
- 26: Projection
- 27: Coupling piece
- 28: Coupling counterpart
- 29: Engagement
- 30: Hook structure
- 31: Arrow direction
- 32: Arrow direction

## Claims

1. Implant (10), in particular a removable implant (10), for dilating an urethra of a person by applying a local ischemic pressure to the tissue of the urethra through a wire structure (11) with at least two wires (12), wherein the implant (10) is insertable into the urethra with a distal end (13) first, **characterized in that** a proximal end (14) of the implant (10) has a coupling piece (15, 27) with a ferromagnetic element (18), wherein the implant (10) is withdrawable from the urethra via this coupling piece (15, 27).

2. Implant (10) according to claim 1, **characterised in that** the ferromagnetic element (18) is a permanent magnet.

3. Implant (10) according to claim 1 or 2, **characterised in that** the ferromagnetic element is the coupling piece (15, 27).

4. Implant (10) according to claim 1 or 2, **characterised in that** the ferromagnetic element (18) is integrated into the coupling piece (15, 27), in particular is enclosed by the coupling piece (15, 27).

5. Implant (10) according to any one of the preceding claims, **characterized in that** the coupling piece (15, 27) comprises at least one projection, slot, notch (19), hole, pin or the like as mechanical coupling means.

6. Implant (10) according to any one of the preceding claims, **characterised in that** the coupling piece (15, 27) has a circular or a circular sectional cross-section.

7. Implant (10) according to one of the previous claims, **characterised in that** a sensor and/or a data memory is arranged in the coupling piece (15, 27), in particular that a sensor and/or a data memory is integrated in the coupling piece (15, 27).

8. Implant (10) according to any one of the preceding claims, **characterised in that** the coupling piece (15, 27) is made of a plastic.

9. Implant (10) according to any one of the preceding claims, **characterised in that** the coupling piece (15, 27) is connected to the wire structure (11) via a wire or thread (16) or directly.

10. Hand-held medical device (20) for positioning and retrieving an implant (10) in or out of an urethra with a rod (22), in particular a flexible rod, which can be introduced into a catheter (21), the rod (22) having a coupling counterpart (24, 28) at a distal end (23) and the implant (10) being designed according to at least one of claims 1 to 9, **characterized in that** the coupling counterpart (24, 28) has a ferromagnetic counter-element (25) for coupling with a coupling piece (15, 27) of the implant (10).

11. Hand-held medical device according to claim 10, **characterised in that** the ferromagnetic counter element is a permanent magnet.

12. Handheld medical device (20) according to claim 10 or 11, **characterised in that** the ferromagnetic counter element (25) is the coupling counter element (24, 28).

13. Hand-held medical device (20) according to claim 10 or 11, **characterized in that** the ferromagnetic counter element (25) is integrated into the coupling counter piece (24, 28), in particular is enclosed by the coupling counter piece (24, 28).

14. Handheld medical device (20) according to any one of claims 10 to 13, **characterized in that** the coupling counterpart (24, 28) comprises at least one projection (26), slot, notch (19), bore, pin or the like as mechanical coupling means, said coupling means being complementary to the coupling means of the coupling piece (15, 27).

15. Handheld medical device (20) according to any one of claims 10 to 14, **characterised in that** the coupling piece (15, 27) of the implant (10) and the coupling counterpart (24, 28) have a common circular or circular cross-section when assembled.

16. Hand-held medical device (20) according to one of claims 10 to 15, **characterized in that** a sensor and/or a data memory is arranged in the coupling counterpart (24, 28), in particular **in that** a sensor and/or a data memory is integrated in the coupling counterpart (24, 28).

17. Hand-held medical device (20) according to any one of claims 10 to 16, **characterized in that** the coupling counterpart (24, 28) is made of a plastic material.
